# EUROPEAN PATENT SPECIFICATION

(11) **EP 3 924 103 B1**
(45) Date of publication and mention of the grant of the patent: **12.11.2025**
(21) Application number: 20724562.2
(22) Date of filing: 13.02.2020
(51) Int. Cl.: B01J 20/06, B01J 20/02, B01J 20/28, B01J 20/32

(54) **A COMPOSITE MATERIAL AND A METHOD TO PREPARE THE COMPOSITE**
VERBUNDWERKSTOFF UND VERFAHREN ZUR HERSTELLUNG DES VERBUNDSTOFFS
MATÉRIAU COMPOSITE ET PROCÉDÉ DE PRÉPARATION DU COMPOSITE

(30) Priority: 13.02.2019 ZA 201900915
(43) Date of publication of application: 22.12.2021
(73) Proprietor: CSIR, 0184 Pretoria (ZA); Mintek, 2194 Randburg (ZA)
(72) Inventor: SIKHWIVHILU, Lucky, 2091 Johannesburg (ZA); HILLIE, Thembela, 0157 Pretoria (ZA)
(74) Representative: Arnold & Siedsma
(86) International application number: PCT/IB2020/051169
(87) International publication number: WO 2020/165812

(56) References cited:
- WO-A1-2013/093805
- US-A- 5 961 843
- US-A1- 2009 147 370
- US-A1- 2009 220 600
- US-A1- 2010 213 046

## Description

### INTRODUCTION

This invention relates to a composite material, comprising an active layer and a substrate layer, and a method to prepare the composite material. The invention further relates to the use of the composite material for reducing contaminants in water.

### BACKGROUND

Contaminated water, containing unacceptable levels of toxic substances and pathogens, is often used by humans for consumption, agricultural irrigation, or recreation. The use of such contaminated water leads to infection and development of disease. The World Health Organization (WHO) recommends that any water intended for drinking should contain zero total faecal coliform counts in any 100 ml sample. Due to poverty and scarcity of resources, the use of contaminated water, not complying to the WHO recommended standard, is still frequently used in many developing countries.

Currently, a number of methods are used for purifying water, including ultraviolet light, low frequency ultrasonic irradiation, distillation, reverse osmosis, water sediment filters, activated carbon, and ozonisation. A disadvantage of these methods is that they require relatively expensive apparatus, placing them out of reach of most of the population exposed to contaminated water.

Furthermore, halogens such as chlorine (CI) and bromine (Br) are well known and widely used as antibacterial agents, but the direct use of these halogens as antibacterial agents has, as a result of their high toxicity and high vapour pressure in pure form, many disadvantages. Another disadvantage associated with the use of halogens is that they may potentially react with other chemical contaminants to form by-products that could be carcinogenic.

Yet another disadvantage of these methods is that some of them, such as those using activated carbon, insufficiently remove contaminants including bacteria, algae and fungi from water. In addition, they may require an extended period of time to sufficiently reduce the contaminants. Another disadvantage experienced with some of the above methods are that they have limited efficiency whilst being costly and time consuming.

United States patent application no. US 2010/0032353 discloses a liquid dispenser or water purification unit having a housing and a mouthpiece configured for contact with the mouth of a person. At least part of the mouthpiece is provided with an antimicrobial surface coating comprising silver nanoparticles complexed with titanium dioxide. A disadvantage associated with US 2010/0032353 is that the filter embedded in the unit lasts only a year, (approximately 700 liters) meaning users must replace the unit annually. A further disadvantage is that the antimicrobial surface is not used for water purification, but to ensure that bacteria from one person holding or drinking from the mouthpiece are killed on contact with the antimicrobial surface, such that a second person using the mouthpiece is not infected by the bacteria. A device according to this invention will not be suitable for decontaminating a body of water.

International patent application publication no. WO 03/092886 discloses a mesoporous TiO₂ thin film, wherein the thin film is prepared from a TiO₂ sol-gel prepared from hydrolysis and condensation of titanium alkoxide in the presence of a stabilizer. A so-called dip coating technique is employed to create a TiO₂ thin film on the substrate. One particular disadvantage associated with the method disclosed for producing the coating on the substrate, is that the bond between the substrate and the nanoparticles cannot be sufficient in ensuring that nanomaterial particles are not leached into water on extended exposure thereto.

International patent application publication no. WO 2013/093805 discloses a method for preparing a nanotubular composite matrix for reducing contaminants in water, the method including the steps of preparing a titanium dioxide in nanotubular form, dissolving silver nanoparticles in a solvent, impregnating the titanium dioxide with the silver nanoparticles in solution to form a matrix, removing excess solvent from the matrix, and calcining the matrix to form a nanotubular composite matrix. The invention further relates to a method for reducing contaminants in water by incubating water exposed to the composite matrix and activating the matrix by exposure to a light source.

However, the disclosure fails to disclose a device or a method that can be used to decontaminate water without the nanomaterial particles being leached into the water. The disclosure further fails to disclose a method, whereby the composite matrix can be applied to a substrate such that, in use, the nanomaterials will not leach into the water being treated, whilst simultaneously maintaining efficiency comparable to that of the nanomaterial precursor material.

Therefore, there remains a need for a robust and effective method to produce a composite material, or a device comprising a composite material, that will ensure that the efficiency of the precursor material is maintained once bonded to the substrate, whilst simultaneously addressing the shortcomings of the prior art in terms of the leaching of the materials into the water being treated.

### SUMMARY OF THE INVENTION

According to a first aspect to the present invention there is provided a composite material suitable for treating a body of water, comprising an active layer and a substrate layer, wherein the active layer comprises titanium dioxide nanotubes and silver nanoparticles, and wherein the active layer is deposited onto and bonded to the substrate layer by providing an active layer solution of silver nanoparticle loaded titanium dioxide nanotubes, aerosolizing the active layer solution and directing the aerosol towards a heated substrate, wherein the substrate is heated to a temperature of from 100 °C to 500 °C, preferably from 150 °C to 300 °C, more preferably to a temperate of 250 °C, thereby depositing the active layer onto the substrate, wherein the substrate is a suitable substrate that can withstand the temperatures to which the substrate will be exposed to during the coating process including glass, stainless steel, and ceramic material, such that, in use, there is substantially no leaching of the active layer into the body of water, and to maintain the chemical structure, including the shape, size, and configuration, of the nanocomposite material once the active layer is deposited and bonded to the substrate layer.

In one embodiment, the active layer has an average thickness of from about 25 nm to about 250 nm, preferably from about 50 nm to about 150 nm, more preferably from about 60 nm to about 85 nm.

According to the invention, the titanium dioxide nanoparticles are titanium dioxide nanotubes.

Preferably, titanium dioxide nanotubes have a cross-sectional diameter of from about 2 nm to about 200 nm, more preferably from about 5 nm to about 100 nm, and most preferably from about 8 nm to about 20 nm.

In one embodiment, the silver nanoparticles have a cross-sectional diameter of from about 0.5 nm to about 30 nm, preferably from about 1 nm to about 10 nm, more preferably from about 2 nm to about 5 nm.

According to a second aspect to the present invention there is provided a method for preparing a composite material comprising an active layer and a substrate layer, the method comprising providing an active layer solution of silver nanoparticle loaded titanium dioxide nanotubes, aerosolizing the solution and directing the aerosol towards a heated substrate, wherein the substrate is heated to a temperature of from 100 °C to 500 °C, preferably from 150 °C to 300 °C, more preferably to a temperate of 250 °C, thereby to deposit the active layer onto the substrate, wherein the substrate is a suitable substrate that can withstand the temperatures to which the substrate will be exposed to during the coating process including glass, stainless steel, and ceramic material.

In one embodiment, the method comprises directing the aerosol through a nozzle towards the heated substrate, thereby to produce a coating spray.

In a preferred embodiment, the active layer solution of silver nanoparticle loaded titanium dioxide nanoparticles is aerosolized by placing the solution in a nebulizer bowl and flowing compressed air through the solution, thereby to form an aerosolized solution.

Preferably, the active layer solution is an aqueous solution.

Preferably, the active layer has an average thickness of from about 25 nm to about 250 nm, more preferably from about 50 nm to about 150 nm, and most preferably from about 60 nm to about 85 nm.

According to the invention, the active layer is deposited and bonded to the substrate such that, when in use in water, the active layer of the composite has a substantially zero leaching rate.

In one embodiment, the active layer solution of silver nanoparticle loaded titanium dioxide nanoparticles has a concentration of Ag/TiO₂ of from about 1% to about 10%, more preferably from about 2% to about 8%, most preferably about 5%.

According to a third aspect of the present invention there is provided a composite material produced according to the method of the invention.

According to a further aspect of the present invention there is provided a device suitable for use in disinfecting a body of the water, the device comprising a composite material according to the invention.

According to a further aspect to the present invention there is provided a method of disinfecting a body of water, the method comprising contacting a body of water with a composite material according to the invention, and exposing the water and composite material to a source of UV irradiation.

### BRIEF DESCRIPTION OF THE DRAWINGS

The invention will now be described in more detail with reference to the following non-limiting embodiments and figures in which:
- **Figure 1**: shows TEM images at 100 nm (a) and 20 nm (b) scale for the Ag/TiO₂ nanocomposite powder;
- **Figure 2**: shows a SEM surface morphology images of Ag/TiO₂ active layers produced using the method of the invention at (a) 150 °C, (b) at 250 °C, (c) at 350 °C, and (d) at 450 °C;
- **Figure 3**: shows Ag/TiO₂ active layer thickness for active layers produced using the method of the invention at (a) 150 °C, (b) at 250 °C, (c) at 350 °C, and (d) at 450 °C; and
- **Figure 4**: shows a graphical representation of the number if *E*. *coli* colonies detected, over time, in various water samples after treatment with the composite of the invention.

### DETAILED DESCRIPTION OF A PREFERRED EMBODIMENT

The present invention will now be described more fully hereinafter with reference to the accompanying figures, in which some of the non-limiting embodiments of the invention are shown.

Although specific terms are employed herein, they are used in a generic and descriptive sense only and not for purposes of limitation.

As used herein, throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having", "including", and variations thereof used herein, are meant to encompass the items listed thereafter, and equivalents thereof as well as additional items.

The present invention provides for a composite material that comprises an active layer and a substrate layer. The active layer comprises a nanocomposite matrix which includes titanium dioxide nanoparticles loaded with silver nanoparticles.

According to the invention the titanium dioxide nanomaterial used in the preparation of the precursor matrix, and ultimately the active layer, is nanotubular titanium dioxide.

The silver loaded titanium dioxide nanocomposite is the precursor for the active layer. The precursor nanocomposite matrix is placed in solution and applied to a substrate as an active. It is of critical importance, according to an object of this invention, that the bonding at the interface between the active layer and the substrate be such that the nanomaterial leaching rate is such that is acceptable, and desirable, for extended exposure of the composite material to a body of water being treated in use. Furthermore, in terms of leaching of these materials into a substance to be consumed, it is not only the bonding between the active layer and the substrate that is important, but also the physical integrity of the active layer as such.

The active layer, comprising the silver loaded titanium dioxide nanomaterial, has to be applied to the substrate in such a manner that it provides a desirable bond and layer integrity, as described above. However, the primary function of the layer, *i.e.* the efficiency thereof in the process of removing contaminants from water, has to be maintained. This efficiency, compared to the efficiency of the nanocomposite precursor material, is integrally linked with maintaining the chemical structure, including the shape, size, and configuration, of the nanocomposite material once it is applied as a layer as part of a composite material.

The active layer, comprising the silver loaded titanium dioxide nanomaterial, is applied to the substrate at an average layer thickness, as measured using to scanning electron microscopy, of about 60 nm to about 85 nm. Preferably, active layer has an average layer thickness of about 25 nm to about 250 nm, about 25 nm to about 240 nm, about 25 nm to about 230 nm, about 25 nm to about 220 nm, about 25 nm to about 210 nm, about 25 nm to about 200 nm, about 25 nm to about 190 nm, about 25 nm to about 180 nm, about 25 nm to about 170 nm, about 25 nm to about 160 nm, about 25 nm to about 150 nm, about 25 nm to about 140 nm, about 25 nm to about 130 nm, about 25 nm to about 120 nm, about 25 nm to about 110 nm, about 25 nm to about 100 nm, about 25 nm to about 90 nm, and about 25 nm to about 85 nm.

Preferably, the active layer has an average layer thickness of about 30 nm to about 250 nm, about 30 nm to about 240 nm, about 30 nm to about 230 nm, about 30 nm to about 220 nm, about 30 nm to about 210 nm, about 30 nm to about 200 nm, about 30 nm to about 190 nm, about 30 nm to about 180 nm, about 30 nm to about 170 nm, about 30 nm to about 160 nm, about 30 nm to about 150 nm, about 30 nm to about 140 nm, about 30 nm to about 130 nm, about 30 nm to about 120 nm, about 30 nm to about 110 nm, about 30 nm to about 100 nm, about 30 nm to about 90 nm, and about 30 nm to about 85 nm. More preferably, the active layer has an average layer thickness of about 40 nm to about 250 nm, about 40 nm to about 240 nm, about 40 nm to about 230 nm, about 40 nm to about 220 nm, about 40 nm to about 210 nm, about 40 nm to about 200 nm, about 40 nm to about 190 nm, about 40 nm to about 180 nm, about 40 nm to about 170 nm, about 40 nm to about 160 nm, about 40 nm to about 150 nm, about 40 nm to about 140 nm, about 40 nm to about 130 nm, about 40 nm to about 120 nm, about 40 nm to about 110 nm, about 40 nm to about 100 nm, about 40 nm to about 90 nm, and about 40 nm to about 85 nm.

Even more preferably, the active layer has an average layer thickness of about 50 nm to about 250 nm, about 50 nm to about 240 nm, about 50 nm to about 230 nm, about 50 nm to about 220 nm, about 50 nm to about 210 nm, about 50 nm to about 200 nm, about 50 nm to about 190 nm, about 50 nm to about 180 nm, about 50 nm to about 170 nm, about 50 nm to about 160 nm, about 50 nm to about 150 nm, about 50 nm to about 140 nm, about 50 nm to about 130 nm, about 50 nm to about 120 nm, about 50 nm to about 110 nm, about 50 nm to about 100 nm, about 50 nm to about 90 nm, and about 50 nm to about 85 nm.

Most preferably, the active layer has an average layer thickness of about 60 nm to about 250 nm, about 60 nm to about 240 nm, about 60 nm to about 230 nm, about 60 nm to about 220 nm, about 60 nm to about 210 nm, about 60 nm to about 200 nm, about 60 nm to about 190 nm, about 60 nm to about 180 nm, about 60 nm to about 170 nm, about 60 nm to about 160 nm, about 60 nm to about 150 nm, about 60 nm to about 140 nm, about 60 nm to about 130 nm, about 60 nm to about 120 nm, about 60 nm to about 110 nm, about 60 nm to about 100 nm, about 60 nm to about 90 nm, and about 60 nm to about 85 nm.

According to the invention the active layer comprises silver nanoparticles loaded onto nanotubular titanium dioxide. The nanotubular titanium dioxide in the active layer, *i.e.* after application and bonding of the active layer to the substrate, have a cross-sectional diameter of about 2 nm to about 200 nm, about 5 nm to about 180 nm, about 5 nm to about 160 nm, about 5 nm to about 150 nm, about 5 nm to about 140 nm, about 5 nm to about 130 nm, about 5 nm to about 120, about 5 nm to about 110 nm, about 5 nm to about 100 nm, about 6 nm to about 90 nm, about 6 nm to about 80 nm, about 6 nm to about 70 nm, about 6 nm to about 60 nm, about 6 nm to about 50 nm, about 6 nm to about 40 nm, about 6 nm to about 30 nm, about 6 nm to about 20 nm, about 7 nm to about 20 nm, about 8 nm to about 20 nm, about 10 nm to about 20 nm.

The silver nanoparticles in the active layer, *i.e.* after application and bonding of the active layer to the substrate, have a cross-sectional diameter of from about 0.5 nm to about 30 nm. Preferably, the silver nanoparticles have a cross-sectional diameter from about 1 nm to about 10 nm, about 1 nm to about 8 nm, about 1 nm to about 7 nm, about 1 nm to about 6 nm about, and about 1 nm to about 2 nm. More preferably, the silver nanoparticles have a cross-sectional diameter of from about 2 nm to about 10 nm, about 2 nm to about 8 nm, about 2 nm to about 7 nm, about 2 nm to about 6 nm, and preferably from about 2 nm to about 5 nm.

The substrate is any suitable substrate that can withstanding the temperatures to which the substrate will be exposed to during the coating process. Suitably substrates include, but are not limited to, glass, stainless steel, and ceramic material.

The present invention further provides for a method for preparing a composite material that comprises an active layer and a substrate layer, the method comprising providing an active layer solution of silver nanoparticle loaded titanium dioxide nanotubes, aerosolizing the solution and directing the aerosol towards a heated substrate, thereby to deposit the active layer onto the substrate.

The active layer solution, comprising the silver loaded titanium dioxide nanotubes, aerosolized by placing the solution in a nebulizer bowl and flowing compressed air through the solution, thereby to form an aerosolized solution. However, an aerosol of the solution may be produced in any other manner, including by ultrasonication.

As used in this specification, the term "aerosol", "aerosolizing" and "aerosolized" should be understood to describe a mixture or a suspension of fine solid particles, and/or liquid particles, in air or any another gas.

The aerosolized solution is directed at the substrate to be coated through a spray nozzle, thereby creating an aerosol spray of the active layer solution. The substrate to be coated glass, stainless steel, or a ceramic type material. In one embodiment of the invention, the active layer was coated onto a glass substrate that was heated to a temperate of about 250 °C.

While a temperature of 250 °C may be preferred for a particular type of glass, it will be understood that this temperature may vary depending on the substrate to be coated. The temperature of the substrate is from about 100 °C to about 500 °C. The temperature of the substrate may be from about 100 °C to about 400 °C, about 100 °C to about 300 °C, or about 100 °C to about 250 °C.

The temperature of the substrate may be from about 150 °C to about 500 °C. In particular, the temperature may be about 150 °C to about 400 °C, about 150 °C to about 300 °C, or about 150 °C to about 250 °C, limits inclusive. For example, the temperature of the substrate may be about 150 °C, about 160 °C, about 170 °C, about 180 °C, about 190 °C, about 200 °C, about 210 °C, about 220 °C, about 230 °C, about 240 °C, or about 250 °C.

The concentration of TiO₂ and Ag in the active layer solution may be about 1% to about 10%, about 2% to about 8%, about 3% to about 6%, or about 5%.

### Preparation of TiO₂ nanotubes, nanorods or nanospheres

The titanium dioxide nanoparticles were also prepared from a commercial P25 Degussa titania powder; the nanotubes are according to the invention. In order to prepare the titanium dioxide nanotubes, about 23 g of the Degussa titania powder was placed in a teflon container together with 150 ml of 18 M potassium hydroxide to form a mixture.

The mixture was stirred at a constant rate of 500 rpm in an autoclave at a temperature of about 150 °C for 24 hours. Alternatively, the mixture was stirred at a constant rate of 500 rpm in a microwave reactor for about 5 - 10 minutes. The mixture was allowed to cool at room temperature for 24 hours to form titanium dioxide nanotubes.

Subsequently, the nanotubes were separated from the mixture by centrifugation at a stirring rate of 10 000 rpm for 30 minutes at about 4 °C. The nanotubes were again washed with deionised water to remove any excess potassium hydroxide from the nanotubes thereby to maintain conductivity of the nanotubes constant below 100 pS/cm.

The nanotubes were dried in an oven at about 120 °C for 12 hours and then allowed to cool to room temperature for 5 hours. The nanotubes were then sieved to a desired size. The nanotubes had a cross-sectional diameter of from about 7 nm to about 11 nm. However, the nanotubes may also have a cross-section diameter of about 2 nm to about 200 nm, about 5 nm to about 180 nm, about 5 nm to about 160 nm, about 5 nm to about 150 nm, about 5 nm to about 140 nm, about 5 nm to about 130 nm, about 5 nm to about 120, about 5 nm to about 110 nm, about 5 nm to about 100 nm, about 6 nm to about 90 nm, about 6 nm to about 80 nm, about 6 nm to about 70 nm, about 6 nm to about 60 nm, about 6 nm to about 50 nm, about 6 nm to about 40 nm, about 6 nm to about 30 nm, about 6 nm to about 20 nm, about 7 nm to about 20 nm, about 8 nm to about 20 nm, about 10 nm to about 20 nm.

The above method as described was also substantially followed for the preparation of the titanium dioxide in a nanorods form, with the exception that 10 M of potassium hydroxide was used.

The titanium dioxide in a nanospherical form was prepared by using the above commercial P25 Degussa titania powder. The powder was modified prior to use. The modification was achieved by mixing the powder with water and then compressing the mixture into a moist paste. This moist paste was then dried in an oven at 120 °C for 48 hours to form the titanium dioxide nanospheres. The nanospheres are cooled and crushed into fragments. The fragments having the desired size of the nanospherical form are sieved out. The larger fragments are recycled and re-sieved while the smaller ones (fine powder formed as a result of crushing) are mixed with water again to from the moist paste to be dried.

### Loading titanium dioxide nanoparticles with silver

Silver nitrate was prepared and loaded onto the titanium dioxide nanotubes, nanorods or nanospheres. About 5% w/w of the silver nitrate was dissolved in 10 ml of deionised water to form a mixture. The mixture was then added to a container containing about 4 g of the nanotubes, nanorods or nanospheres to form a matrix.

Excess water was removed by drying the matrix at room temperature for 24 hours and subsequently in an oven at 100 °C for a further period of 12 hours. It was observed that the concentration of the loaded silver nitrate differs based on the nature of the TiO₂ form used. In all instances, nitrate was used as a precursor, and water was used as a solvent due to excellent solubility of the nitrate in water. The matrix was calcined at 200 °C for 5 hours to remove the nitrate moiety from the silver nitrate to form a composite matrix (nanotubular, nanorod, or nanospherical) material to be uses as a precursor in the formation of the active layer.

### Synthesis of composite material comprising Ag/TiO₂ nanocomposite active layer

In the method according to the present invention, the Ag/TiO₂ nanocomposite, prepared according to the method described above, was placed into solution, which solution was aerosolized and sprayed through a coating nozzle onto a heated surface.

The apparatus that was used for aerosolizing and spraying the solution is comprises a spray unit, a liquid feeding unit, and a temperature control unit (Carbolite furnace). The spray unit was similar to that used by asthma patients in the delivery of pharmaceutical products. The spray unit consists of a compressor, a nebulizer bowl, and tubing.

To achieve deposition, the Ag/TiO₂ nanocomposite solution was placed in the nebulizer bowl, with the bowl and the compressor being connected through the required tubing. The compressor provides compressed air that compresses the liquid precursor into a fine mist or aerosol. The aerosolized active layer solution, comprising the Ag/TiO₂ nanoparticles, flows through the tubing towards the coating nozzle via a globe valve, which is used to control the flow of the aerosol to the heated substrate. The aerosolized active layer solution flows across the tubing to the furnace, where the glass substrate to be coated was placed inside a glass tube.

In order to obtain optimal characteristics of the active layer, parameters such as substrate temperature, layer thickness and other deposition conditions were investigated. To optimize the synthesis of the active layer, in terms of bonding and maintaining the characteristics of the composite matrix precursor material, substrate temperatures were varied from 150 °C to 450 °C (150 °C, 250 °C, 350 °C and 450 °C). Application time of the aerosol spray to the substrate was set at 1 hour. It was found that the active layer thickness depends, in part, on the position of the substrate in the furnace.

### Active layer characterisation

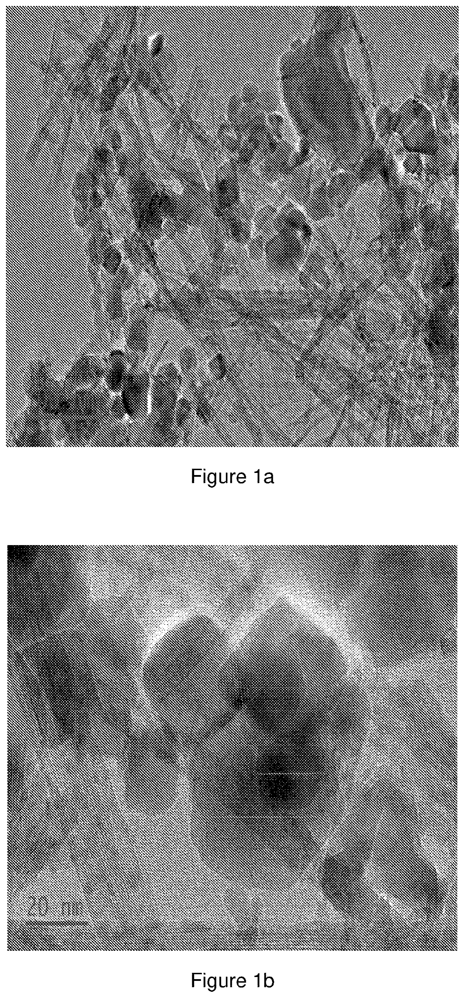

Transmission Electron Microscopy (TEM) was performed using a JEOL 2010 high-resolution transmission electron microscope with a LaB₆- cathode operated at a voltage of 200kV on the Ag/TiO₂ nanocomposite powder. As can be seen from the TEM images in Figure 1, both image (a) and (b) shows the distribution of silver nanoparticles and titanium dioxide nanotubes. The TEM images also show that silver nanoparticles are found on the surface of titanium dioxide nanotubes (Figure 1b, scale 20nm). The silver nanoparticles found on the surface of the titanium dioxide nanotubes has an average diameter of about 2 - 5nm. Further, as can be seen from Figure 1, the titanium dioxide nanotubes were needle-shaped with an average cross-sectional diameter ranging from about 8 - 20nm.

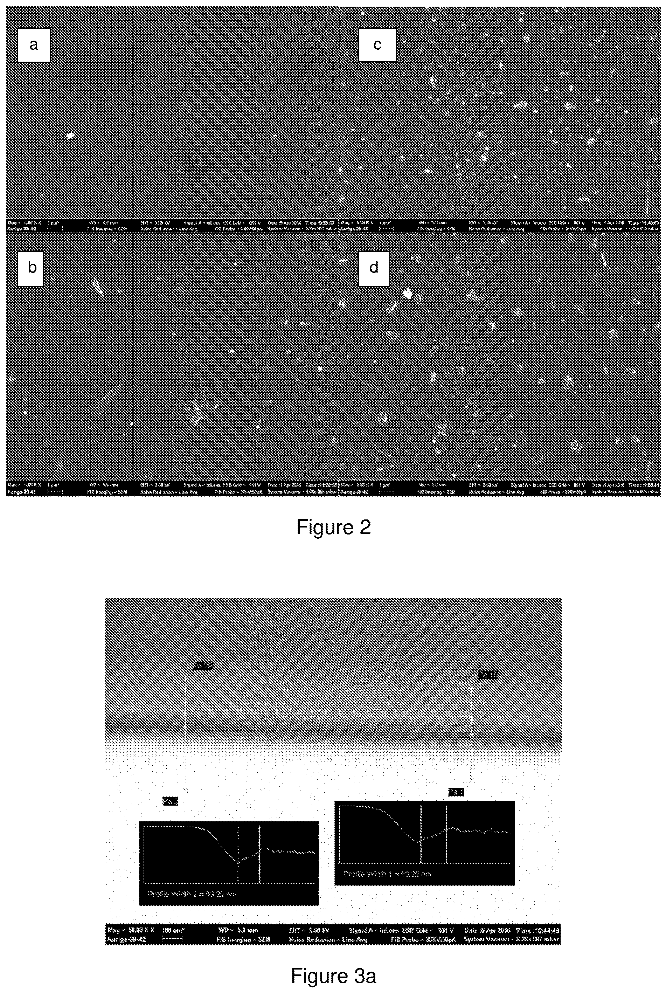

Figure 2 shows scanning electron microscopy (SEM) images of the active layers produced at various temperatures. The surface morphology of the Ag/TiO₂ nanomaterial containing active layers were investigated by Joel SEM (JSM-5800 LV, JEOL) at 5000x magnification.

During deposition, the surface energy of the glass substrate is higher than the surface tension of the solution. Droplets tend to blast on the glass substrate creating a uniform deposition of particles. This process may be referred to as wetting. As the thickness increases, surface energy tends to be lower than the surface tension on top of the deposited active layer. This leads to the formation of particles on top of the active layer.

Figure 2a shows the active layer deposited at 150 °C, and it can be seen that the layer's surface consists of small and non-uniform crystallites. The surface has a sparsity of particles. Fig 2b shows the active layer deposited at 250 °C. It can be observed that particles in this temperature start to adhere on top of the layer when the solution is allowed to evaporate and deposit onto the layer, thickness tends to increase. As the temperature increases from 250 °C to 350 °C, and to 450 °C, as is shown in Figures 2b-2d respectively, more particles are observed with varying particle size and shape.

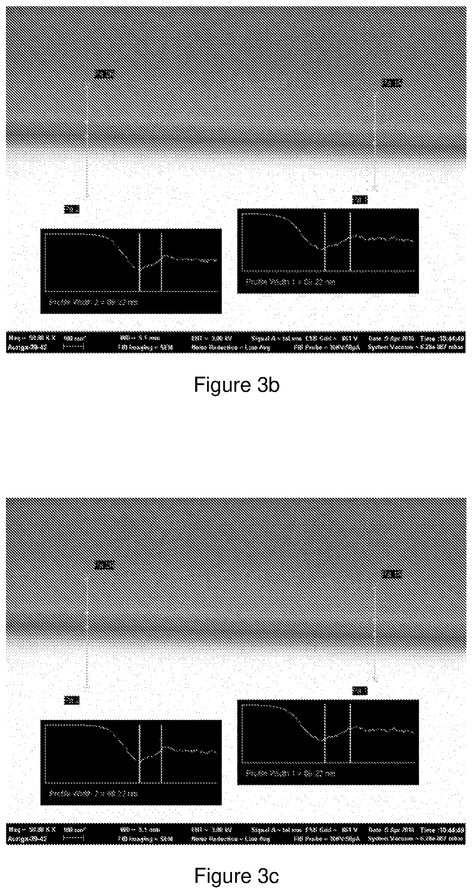

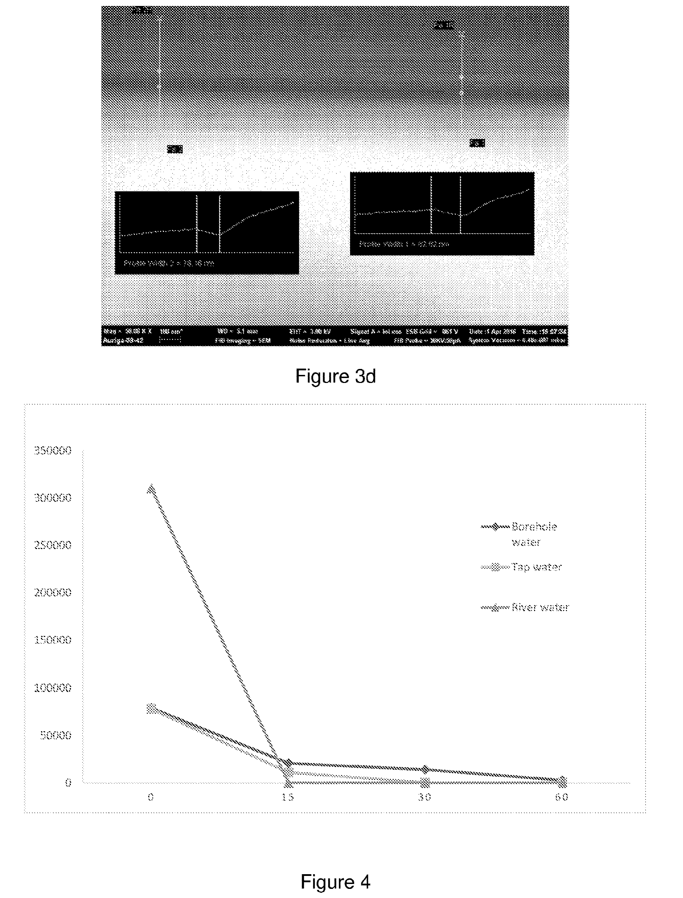

In order to confirm that an active layer was produced, thickness was measured with JEOL SEM (JSM-5800 LV, JEOL) using a cross sectional method. As can be seen in Figure 3, the average active layer thickness increased linearly with increasing substrate temperature. Without thereby wishing to be bound by any particular theory, it is belied that, during aerosol spray deposition, droplets reach the heated substrates and undergo adhesion, suggesting that at higher temperatures (250 °C, 350 °C and 450 °C) the rate of evaporation of the solution is much faster, which results in an increase inlayer thickness. This can also be seen from Figure 2 that particles on the surface of the layer at high temperatures become uniform. The active layer produced at a temperature of 150 °C (Figure 2a), which has particles sparsely on its surface morphology, has an average thickness of 62.2 nm (Figure 3a).

The active layer produced at 250 °C, shown in Figure 2b, has an average thickness of 73.69 nm (Figure 3b). Compared to the film produced at 150 °C, it can be seen that the temperature has an influence on the active layer thickness. In Figure 3c, the active layer has an average thickness of 82.62 nm, while in Figure 3d the active layer also has an average thickness of 82.62 nm.. This would appear to suggest that as the temperature increases, the active layer thickness increases, but only up to a certain optimised temperature point. Since the surface energy of the substrate becomes lower than the surface tension of the droplets, thickness tends to be constant but the formation of particulates tends to increase. This can be seen in Figures 3c and 3d respectively.

### Efficacy Studies - Antibacterial tests

*Escherichia coli* (*E. coli,* ATCC 15597) and salmonella was selected for antibacterial testing of the composite material of the invention. Three water sample types, namely river water, borehole water and tap water, were filtered and tested before dosing with known amounts of *E. coli* and salmonella, thereby to determine the number of colonies present.

Three water samples were dosed with *E. coli* and salmonella then filtered to determine the number of *E. coli* and salmonella colonies present. This was done for a comparison of the number of colonies present prior to and after treatment.

After dosing with *E. coli* (2.5 µL of *E. coli* ATCC15597), water samples (12 ml each) were left to stand in the sun with a composite material according to the invention, produced at 250 °C, added to each sample. The samples were left to stand in the sun for 15 minutes, 30 minutes, and 60 minutes respectively. Three samples were tested for each water type. After exposure, the samples were tested for the number of *E*. *coli* colonies. A similar process was followed for salmonella testing.

The method used to measure microbial count was the Heterotropic Count Microbial Indicator method (as described in Bortram *et al.,* 2003). This method was used to monitor the performance disinfection process.

Referring now to Figure 4, on average, after exposure of 15 minutes to the composite material of the invention a reduction of 74%, 86%, and 99% were seen for the borehole water, tap water and river water samples, respectively. After 30 minutes of exposure, a 99% reduction was seen for tap water, and after 60 minutes an 81% reduction for borehole water samples.

In addition to the above efficiency tests, a cycle test was conducted to determine whether the active layer according to the present invention would maintain its efficiency over an extended period of time. During the cycle test, 2L of each water samples were initially tested to determine the level of *E. coli* present. The water samples (borehole, tap and river water) were dosed with 2.5 µL of *E. coli* ATCC15597, after which samples were split into 5 separate 120ml samples and labelled "Cycle 1" to "Cycle 5".

Composite material comprising an active layer of silver loaded titanium dioxide nanomaterial, produced at 250 °C, was first inserted into each water sample labelled Cycle 1, and left for 60 minutes. After 60 minutes, composite material was transferred to the next sample labelled Cycle 2 and left for 60 minutes. This procedure was repeated up to the last sample, labelled Cycle 5, for each of the three water sample types. Results obtained for the different water types, dosed with *E. coli,* are shown in Table 1 below.

**Table 1: Number of E. coli colonies present after 60 minutes per cycle for various water samples.**

| **Water sample** | **After spiking** | **Cycle 1** | **Cycle 2** | **Cycle 3** | **Cycle 4** | **Cycle 5** |
|---|---|---|---|---|---|---|
| **Tap** | 470 000 | <1 | <1 | <1 | <1 | <1 |
| **Borehole** | 420 000 | 2000 | 110 | 14 | <1 | <1 |

## Claims

1. A composite material suitable for treating a body of water, comprising an active layer and a substrate layer, wherein the active layer comprises titanium dioxide nanotubes loaded with silver nanoparticles, and wherein the active layer is deposited onto and bonded to the substrate layer by providing an active layer solution of silver nanoparticle loaded titanium dioxide nanotubes, aerosolizing the active layer solution and directing the aerosol towards a heated substrate, wherein the substrate is heated to a temperature of from 100 °C to 500 °C, preferably from 150 °C to 300 °C, more preferably to a temperate of 250 °C, thereby depositing the active layer onto the substrate, wherein the substrate is a suitable substrate that can withstand the temperatures to which the substrate will be exposed to during the coating process including glass, stainless steel, and ceramic material,
and, such that, in use, there is substantially no leaching of the active layer into the body of water, and to maintain the chemical structure, including the shape, size, and configuration, of the nanocomposite material once the active layer is deposited and bonded to the substrate layer.

2. A composite material according to claim 1, wherein the active layer has an average thickness of from 25 nm to 250 nm, preferably from 50 nm to 150 nm, more preferably from 60 nm to 85 nm, as measured by Scanning Electron Microscope (SEM).

3. A composite material according to claim 1 or claim 2, wherein the titanium dioxide nanotubes in the active layer, after application and bonding of the active layer to the substrate, have a cross-sectional diameter of from 2 nm to 200 nm, more preferably from 5 nm to 100 nm, and most preferably from 8 nm to 20 nm, as measured by Transmission Electron Microscopy (TEM).

4. A composite material according to any one of the preceding claims, wherein the loaded silver nanoparticles in the active layer, after application and bonding of the active layer to the substrate, have a cross-sectional diameter of from 0.5 nm to 30 nm, preferably from 1 nm to 10 nm, more preferably from 2 nm to 5 nm, as measured by Transmission Electron Microscopy (TEM).

5. A method for preparing a composite material comprising an active layer and a substrate layer, the method comprising providing an active layer solution of silver nanoparticle loaded titanium dioxide nanotubes, aerosolizing the active layer solution and directing the aerosol towards a heated substrate, wherein the substrate is heated to a temperature of from 100 °C to 500 °C, preferably from 150 °C to 300 °C, more preferably to a temperate of 250 °C, thereby to deposit the active layer onto the substrate, wherein the substrate is a suitable substrate that can withstand the temperatures to which the substrate will be exposed to during the coating process including glass, stainless steel, and ceramic material.

6. A method according to claims 5, wherein the method comprises directing the aerosol through a nozzle towards the heated substrate, thereby to produce a coating spray.

7. A method according to claim 5 or claim 6, wherein the active layer solution of silver nanoparticle loaded titanium dioxide nanotubes is aerosolized by placing the solution in a nebulizer bowl and flowing compressed air through the solution, thereby to form an aerosolized solution.

8. A method according to any one of claims 5 to 7, wherein the active layer solution is an aqueous solution.

9. A method according to any one of claims 5 to 8, wherein the active layer has an average thickness of from 25 nm to 250 nm, more preferably from 50 nm to 150 nm, and most preferably from 60 nm to 85 nm, as measured by Scanning Electron Microscope (SEM).

10. A method according to any one of claims 5 to 8, wherein the active layer is deposited and bonded to the substrate such that, when in use in water, the active layer of the composite has a substantially zero leaching rate, and to maintain the chemical structure, including the shape, size, and configuration, of the nanocomposite material once the active layer is deposited and bonded to the substrate layer.

11. A method according to any one of claims 5 to 10, wherein the active layer solution of silver nanoparticle loaded titanium dioxide nanotubes has a concentration of Ag/TiO₂ of from 1% to 10%, more preferably from 2% to 8%, most preferably 5%.

12. A device suitable for use in a method of disinfecting a body of water, the device comprising a composite material according to any one of claims 1 to 4.

13. A method of disinfecting a body of water, the method comprising contacting the body of water with a composite material according to any one of claims 1 to 4, and exposing the water and composite material to a source of UV irradiation.

## Patentansprüche

1. Verbundmaterial, das zum Behandeln eines Gewässers geeignet ist, umfassend eine aktive Schicht und eine Substratschicht, wobei die aktive Schicht Titandioxid-Nanoröhren umfasst, die mit Silbernanopartikeln beladen sind, und wobei die aktive Schicht auf der Substratschicht abgeschieden und mit dieser verbunden wird, durch Bereitstellen einer Lösung der aktiven Schicht aus mit Silbernanopartikeln beladenen Titandioxid-Nanoröhren, was die Lösung der aktiven Schicht vernebelt und das Aerosol auf ein erhitztes Substrat richtet, wobei das Substrat auf eine Temperatur von 100 °C bis 500 °C, vorzugsweise von 150 °C bis 300 °C, mehr bevorzugt auf 250 °C erhitzt wird, wodurch die aktive Schicht auf das Substrat abgeschieden wird, wobei das Substrat ein geeignetes Substrat ist, das den Temperaturen standhalten kann, denen das Substrat während des Beschichtungsprozesses ausgesetzt sein wird, einschließlich Glas, Edelstahl und Keramikmaterial, und derart, dass bei Verwendung im Wesentlichen kein Auswaschen der aktiven Schicht in das Gewässer erfolgt, und um die chemische Struktur, einschließlich der Form, der Größe und der Konfiguration, des Nanoverbundmaterials zu erhalten, sobald die aktive Schicht abgeschieden und mit der Substratschicht verbunden ist.

2. Verbundmaterial nach Anspruch 1, wobei die aktive Schicht eine durchschnittliche Dicke von 25 nm bis 250 nm, vorzugsweise von 50 nm bis 150 nm, mehr bevorzugt von 60 nm bis 85 nm, gemessen durch Rasterelektronenmikroskop (SEM), aufweist.

3. Verbundmaterial nach Anspruch 1 oder 2, wobei die Titandioxid-Nanoröhren in der aktiven Schicht nach einem Aufbringen und Verbinden der aktiven Schicht mit dem Substrat einen Querschnittsdurchmesser von 2 nm bis 200 nm, mehr bevorzugt von 5 nm bis 100 nm und am meisten bevorzugt von 8 nm bis 20 nm, gemessen durch Transmissionselektronenmikroskopie (TEM), aufweisen.

4. Verbundmaterial nach einem der vorstehenden Ansprüche, wobei die geladenen Silbernanopartikel in der aktiven Schicht nach dem Aufbringen und Verbinden der aktiven Schicht mit dem Substrat einen Querschnittsdurchmesser von 0,5 nm bis 30 nm, vorzugsweise von 1 nm bis 10 nm, mehr bevorzugt von 2 nm bis 5 nm, gemessen durch Transmissionselektronenmikroskopie (TEM), aufweisen.

5. Verfahren zum Herstellen eines Verbundmaterials, umfassend eine aktive Schicht und eine Substratschicht, das Verfahren umfassend das Bereitstellen einer Lösung der aktiven Schicht aus mit Silbernanopartikeln beladenen Titandioxid-Nanoröhren, was die Lösung der aktiven Schicht vernebelt und das Aerosol auf ein erhitztes Substrat richtet, wobei das Substrat auf eine Temperatur von 100 °C bis 500 °C, vorzugsweise von 150 °C bis 300 °C, mehr bevorzugt auf eine Temperatur von 250 °C erhitzt wird, um dadurch die aktive Schicht auf das Substrat abzuscheiden, wobei das Substrat ein geeignetes Substrat ist, das den Temperaturen standhalten kann, denen es während des Beschichtungsprozesses ausgesetzt sein wird, einschließlich Glas, Edelstahl und Keramikmaterial.

6. Verfahren nach Anspruch 5, wobei das Verfahren das Richten des Aerosols durch eine Düse auf das erhitzte Substrat umfasst, um dadurch ein Beschichtungsspray zu erzeugen.

7. Verfahren nach Anspruch 5 oder 6, wobei die Lösung der aktiven Schicht aus mit Silbernanopartikeln beladenen Titandioxid-Nanoröhren vernebelt wird, durch Platzieren der Lösung in eine Zerstäuberschale und Strömenlassen von Druckluft durch die Lösung, um dadurch eine vernebelte Lösung auszubilden.

8. Verfahren nach einem der Ansprüche 5 bis 7, wobei die Lösung der aktiven Schicht eine wässrige Lösung ist.

9. Verfahren nach einem der Ansprüche 5 bis 8, wobei die aktive Schicht eine durchschnittliche Dicke von 25 nm bis 250 nm, mehr bevorzugt von 50 nm bis 150 nm und am meisten bevorzugt von 60 nm bis 85 nm, gemessen durch Rasterelektronenmikroskop (SEM), aufweist.

10. Verfahren nach einem der Ansprüche 5 bis 8, wobei die aktive Schicht derart abgeschieden und mit dem Substrat verbunden wird, dass die aktive Schicht des Verbundwerkstoffs bei Verwendung in Wasser eine Auswaschrate von im Wesentlichen Null aufweist und um die chemische Struktur, einschließlich der Form, der Größe und der Konfiguration, des Nanoverbundmaterials zu erhalten, sobald die aktive Schicht abgeschieden und mit der Substratschicht verbunden ist.

11. Verfahren nach einem der Ansprüche 5 bis 10, wobei die Lösung der aktiven Schicht aus mit Silbernanopartikeln beladenen Titandioxid-Nanoröhren eine Konzentration von Ag/TiO₂ von 1 % bis 10 %, mehr bevorzugt von 2 % bis 8 %, am meisten bevorzugt 5 % aufweist.

12. Vorrichtung, die zur Verwendung in einem Verfahren zum Desinfizieren eines Gewässers geeignet ist, die Vorrichtung umfassend ein Verbundmaterial nach einem der Ansprüche 1 bis 4.

13. Verfahren zum Desinfizieren eines Gewässers, das Verfahren umfassend ein Inkontaktbringen des Gewässers mit einem Verbundmaterial nach einem der Ansprüche 1 bis 4 und das Aussetzen des Wassers und des Verbundmaterials einer UV-Strahlungsquelle.

## Revendications

1. Matériau composite adapté au traitement d'un plan d'eau, comprenant une couche active et une couche de substrat, dans lequel la couche active comprend des nanotubes de dioxyde de titane chargés de nanoparticules d'argent, et dans lequel la couche active est déposée sur et collée à la couche de substrat en fournissant une solution de couche active de nanotubes de dioxyde de titane chargés de nanoparticules d'argent, en pulvérisant la solution de couche active et en orientant l'aérosol vers un substrat chauffé, dans lequel le substrat est chauffé à une température allant de 100 °C à 500 °C, de préférence de 150 °C à 300 °C, de préférence encore à une température de 250 °C, déposant ainsi la couche active sur le substrat, dans lequel le substrat est un substrat approprié qui peut supporter les températures auxquelles il sera exposé pendant le processus de revêtement, notamment le verre, l'acier inoxydable et les matériaux céramiques, et tel que, lors de l'utilisation, il n'y ait sensiblement pas de lixiviation de la couche active dans le plan d'eau, et afin de maintenir la structure chimique, y compris la forme, la taille et la configuration, du matériau nanocomposite une fois la couche active déposée et collée à la couche de substrat.

2. Matériau composite selon la revendication 1, dans lequel la couche active présente une épaisseur moyenne de 25 nm à 250 nm, de préférence de 50 nm à 150 nm, de préférence encore de 60 nm à 85 nm, telle que mesurée au microscope électronique à balayage (MEB).

3. Matériau composite selon la revendication 1 ou la revendication 2, dans lequel les nanotubes de dioxyde de titane dans la couche active, après application et collage de la couche active sur le substrat, présentent un diamètre en coupe transversale de 2 nm à 200 nm, de préférence de 5 nm à 100 nm, et le plus préférablement de 8 nm à 20 nm, tel que mesuré par microscopie électronique à transmission (TEM).

4. Matériau composite selon l'une quelconque des revendications précédentes, dans lequel les nanoparticules d'argent chargées dans la couche active, après application et collage de la couche active sur le substrat, présentent un diamètre en coupe transversale de 0,5 nm à 30 nm, de préférence de 1 nm à 10 nm, de préférence encore de 2 nm à 5 nm, tel que mesuré par microscopie électronique à transmission (MET).

5. Procédé de préparation d'un matériau composite comprenant une couche active et une couche de substrat, le procédé comprenant la fourniture d'une solution de couche active de nanotubes de dioxyde de titane chargés de nanoparticules d'argent, la pulvérisation de la solution de couche active et l'orientation de l'aérosol vers un substrat chauffé, dans lequel le substrat est chauffé à une température allant de 100 °C à 500 °C, de préférence de 150 °C à 300 °C, de préférence encore à une température de 250 °C, afin de déposer la couche active sur le substrat, dans lequel le substrat est un substrat approprié qui peut supporter les températures auxquelles le substrat sera exposé au cours du processus de revêtement, notamment le verre, l'acier inoxydable et les matériaux céramiques.

6. Procédé selon la revendication 5, dans lequel le procédé comprend l'orientation de l'aérosol à travers une buse vers le substrat chauffé, afin de produire une pulvérisation de revêtement.

7. Procédé selon la revendication 5 ou la revendication 6, dans lequel la solution de couche active de nanotubes de dioxyde de titane chargés de nanoparticules d'argent est pulvérisé en plaçant la solution dans un bol de nébulisation et en faisant circuler de l'air comprimé à travers la solution, afin de former une solution aérosolisée.

8. Procédé selon l'une quelconque des revendications 5 à 7, dans lequel la solution de couche active est une solution aqueuse.

9. Procédé selon l'une quelconque des revendications 5 à 8, dans lequel la couche active présente une épaisseur moyenne de 25 nm à 250 nm, de préférence de 50 nm à 150 nm, et le plus préférablement de 60 nm à 85 nm, telle que mesurée au microscope électronique à balayage (MEB).

10. Procédé selon l'une quelconque des revendications 5 à 8, dans lequel la couche active est déposée et collée au substrat de telle sorte que, lorsqu'elle est utilisée dans l'eau, la couche active du composite présente un taux de lixiviation pratiquement nul, et pour maintenir la structure chimique, y compris la forme, la taille et la configuration, du matériau nanocomposite une fois que la couche active est déposée et collée à la couche de substrat.

11. Procédé selon l'une quelconque des revendications 5 à 10, dans lequel la solution de couche active de nanotubes de dioxyde de titane chargés de nanoparticules d'argent présente une concentration en Ag/TiO₂ de 1 % à 10 %, de préférence encore de 2 % à 8 %, et le plus préférablement de 5 %.

12. Dispositif utilisable dans un procédé de désinfection d'un plan d'eau, le dispositif comprenant un matériau composite selon l'une quelconque des revendications 1 à 4.

13. Procédé de désinfection d'un plan d'eau, le procédé comprenant la mise du plan d'eau en contact avec un matériau composite selon l'une quelconque des revendications 1 à 4, et l'exposition de l'eau et du matériau composite à une source d'irradiation UV.
